# EUROPÄISCHE PATENTSCHRIFT

(11) **EP 0 702 957 B1**
(45) Veröffentlichungstag und Bekanntmachung des Hinweises auf die Patenterteilung: **02.12.1998**
(21) Anmeldenummer: 95114716.4
(22) Anmeldetag: 19.09.1995
(51) Int. Cl.: A61K 35/78, A61K 47/32

(54) **Johanniskrautextrakte**
Extracts of St.John's wort
Extraits d'herbe de la Saint-Jean

(30) Priorität: 24.09.1994 DE 4434170
(43) Veröffentlichungstag der Anmeldung: 27.03.1996
(73) Patentinhaber: Krewel Meuselbach GmbH, 53783 Eitorf (DE)
(72) Erfinder: Schierstedt, Detlef, Dr., D-53757 St. Augustin/Meindorf (DE)
(74) Vertreter: Jönsson, Hans-Peter, Dr.Dipl.-Chem.

(56) Entgegenhaltungen:
- PHARM. IND., Bd. 42, Nr. 10, 1980 Seiten 1009-1018, H.P.MERKLE 'Bildung und Charackterisierung übersättigter Lösungen aus Einbettungen von Arzneistoffen in Polyvinylpyrrolidon'
- PHARM. ACTA HELV., Bd. 61, Nr. 12, 1986 Seiten 337-341, YASUO NOZAWA ET AL. 'Increases in Dissolution Rate of Nifedipine by Roll Mixing with Polyvinylpyrrolidone'
- PHARMAZIE, Bd. 35, Nr. 5/6, - 1980 Seiten 311-312, R.VOIGT ET AL. 'Granulometrische Erfassung des Einflusses von Polyvinylpyrrolidon auf die Lösungsgeschwindigkeit schwerlöslicher Arzneistoffe'
- DATABASE WPI Section Ch, Week 8223 Derwent Publications Ltd., London, GB; Class A96, AN 82-010627 & RO-A-78 402 ( MIRAJ INTR PROD COS) , 28.Februar 1982

## Beschreibung

Gegenstand der Erfindung sind peroral applizierbare Johanniskrautextrakte und ein Verfahren zur Ihrer Herstellung.

Aus Römpp Chemie Lexikon, 9. Auflage, Seite 2099, (1990) ist zu entnehmen, daß Johanniskraut seit alters her gegen Gallen-, Blasen-, Leber- und Magenbeschwerden, Kopfweh, Gicht, Rheuma, als mildes Sedativum, zur Wundheilung arzneilich gebraucht wird, wovon Namen wie Wund-, Wurm-, Sonnwend-, Gottesgnaden-, Herrgottswunder, Hexen-, Bocks-, Teufelsfluchtkraut, Elfenblut, Mannskraft und andere zeugen. Die Pflanze enthält bis 1 % etherisches Öl mit α-Pinen, Myrcen, Cadinen, Gurjunen und Isovaleriansäureestern, außerdem (besonders in den Blüten) die Flavonoide, Quercetin, sein 3-Galactosid (Hyperin) und Rutin sowie Quercitrin und Isoquercitrin. Arzneilich genutzt werden das zur Blütezeit gesammelte Kraut als Tee oder in Form daraus gewonnener Tinkturen sowie das durch Ölauszug (z. B. mittels Olivenöl) aus den frischen Blüten gewonnene tiefrote klare Johanniskrautöl, das ca. 0,004 % Hypericin enthält.

Präparate die Johanniskraut (Herba Hyperici) enthalten, werden innerlich bei psychovegetativen Störungen, depressiven Verstimmungszuständen, Angst und nervösen Unruhezuständen angewendet.

Üblicherweise werden Tees, Tropfen und feste Formen wie Kapseln, Dragees und Filmtabletten beziehungsweise Tabletten eingesetzt. Hauptinhaltsstoffe, welche wesentlich das wirksame Prinzip darstellen sind die Dianthrone darunter das Hypericin.

Nach dem neuesten Deutschen Arzneimittelcode (DAC-Methode) werden die Dianthrone zusammen bestimmt und als Hypericin berechnet. Die Dianthrone sind in Wasser nur schwerstlöslich. Daher ist es hocherwünscht, Extrakte, insbesondere für feste Darreichungsformen einzusetzen, welche die wirksamen Bestandteile im Magen-Darmtrakt bei oraler Einnahme optimal freisetzen.

Die DE 42 39 959 A1 betrifft einen Trockenextrakt aus dem Kraut von Hypericum perforatum L. (Johanniskraut), der durch einen gegenüber dem Hypericingehalt der als Ausgangsmaterial eingesetzten Droge verminderten Hyericingehalt gekennzeichnet ist, wobei der Hypericingehalt vorzugsweise weniger als 0,1 Gew.-% beträgt. Die Erfindung betrifft ferner ein Verfahren zur Herstellung des Trockenextrakts, die Verwendung des Trockenextrakts zur Herstellung psychovegetativ und antidepressiv wirksamer Arzneimittel sowie diese Arzneimittel selbst.

Die DE 42 01 172 C1 betrifft Aloe Vera-Extrakt enthaltende Pellets, die durch eine Dispersion des Aloe Vera-Extrakts in einer Matrix, die vorwiegend aus einem Gerüstbildner, nämlich Kollagen, Gelatine, fraktionierter Gelatine, einem Kollagenhydrolysat und/oder einem Gelatinederivat besteht, gebildet werden.

Die Aufgabe der vorliegenden Erfindung besteht daher darin, peroral applizierbare Johanniskrautextrakte zur Verfügung zu stellen, die eine hohe Freisetzungsrate der wirksamen Bestandteile im Magen-Darmtrakt aufweisen.

In einem ersten Aspekt der vorliegenden Erfindung wird die vorgenannte Aufgabe gelöst durch peroral applizierbare Johanniskrautextrakte, die dadurch gekennzeichnet sind, daß die nicht flüchtige Phase des Extraktes an Polyvinylpyrrolidon in mikrodisperser Form und/oder feste Lösung gebunden, gegebenenfalls neben weiteren Hilfs- und/oder Zusatzstoffen enthalten, wobei das Gewichtsverhältnis von nichtflüchtigen Extraktbestandteilen, insbesondere Spissum oder Siccumphase zu Polyvinylpyrrolidon 1 : 0,4 bis 1 : 5 beträgt.

Im Stand der Technik wurde bisher bei Reinsubstanzen, d. h. chemisch definierten Substanzen, bereits die Adsorption oder Absorption an Polyvinylpyrrolidon vorgeschlagen.

Bei Naturstoffextrakten wie dem Johanniskrautextrakt, der eine Vielzahl von wirksamen Bestandteilen und weiteren Begleitstoffen enthalten, war es nicht zu erwarten, daß ein derart inhomogenes Substanzgemisch eine hohe Löslichkeitsverbesserung mit sich bringt.

Überraschenderweise wurde gefunden, daß durch Lösen des Polyvinylpyrrolidons in einem Johanniskrautfluidextrakt nach dem Einengen ein Extrakt-Träger-Komplex entsteht, bei dem insbesondere die Dianthrone in mikrodisperser Form und/oder in Lösung gebunden sind. Bei der flüssigen Johanniskrautextraktphase handelt es sich insbesondere um eine Spissum- oder Siccumphase, die unter anderem Dianthrone als wirksame Bestandteile enthält.

Bei Messungen der Freisetzung des Extraktes unter Einsatz eines Mediums, das dem Magen-Darm-Milieu entspricht, wurde eine gegenüber dem Stand der Technik signifikant verbesserte Freisetzung der Dianthrone erreicht. Die Wirkstoffreisetzung aus festen peroralen Arzneiformen kann beispielsweise nach dem Deutschen Arzneibuch DAB 10 V5.4 bestimmt werden.

In einer bevorzugten Ausführungsform der vorliegenden Erfindung ist der bei Raumtemperatur feste Träger Polyvinylpyrrolidon mit einem K-Wert von 12 bis 30. Der K-Wert steht in Beziehung zu dem mittleren Molekulargewicht des Polyvinylpyrrolidons. Beispielsweise entspricht ein K-Wert von 15 einem mittleren Molekulargewicht von 10000 während ein K-Wert von 30 einem mittleren Molekulargewicht von 40000 entspricht. Der K-Wert kann auch durch Messungen der Viskosität unter Standardbedingungen gemessen werden. Dieses, im Handel erhältliche weiße bis gelblich-weiße, feine bis körnige Pulver, das als Verdickungsmittel, Suspensionsstabilisator, Lösungsvermittler und Bindemittel für orale Präparate bekannt ist, ist geeignet, feste Lösungen und/oder Mikrodispersionen mit der nicht flüchtigen Extraktphase zu bilden.

Bevorzugterweise wird das Gewichtsverhältnis der nicht flüchtigen Extraktphase zu Polyvinylpyrrolidon im Bereich von 1 : 0,7 bis 1 : 1,5 eingestellt. Wird das Verhältnis von Extrakt zu Polyvinylpyrrolidon zu groß gewählt, so ist die Bindung des Extraktes an Polyvinylpyrrolidon nicht ausreichend und damit keine optimale Freisetzung der wirksamen Bestandteile gegeben.

Zur Herstellung von Tabletten, Filmtabletten, Dragees oder Kapseln werden die peroral applizierbaren Johanniskrautextrakte gegebenenfalls mit weiteren Hilfs- und/oder Zusatzstoffen versetzt. So können bei der Herstellung von Tabletten die Hilfs- und/oder Zusatzstoffe vorzugsweise ausgewählt sein aus Bindemitteln, Gleitmitteln, Füllstoffen, Emulgatoren, Netzmitteln und/oder Sprengmitteln.

Ein weiterer Aspekt der vorliegenden Erfindung umfaßt das Verfahren zur Herstellung von peroral applizierbaren Johanniskrautextrakten mit verbesserter Freisetzung.

Zum Einsatz kommen vorzugsweise Fluidextrakte. Zu diesen wird Polyvinylpyrrolidon gegebenenfalls bei erhöhter Temperatur, zugegeben. Bei erhöhter Temperatur und/oder unter Vakuum wird der Fluidextrakt anschließend bis zur Trockne eingeengt. Fluidextrakte im Sinne der Erfindung umfassen auch Spissum- oder Trockenextrakte, die nach an sich bekannten Verfahren hergestellt werden und durch geeignete Lösungsmittel wieder ganz oder teilweise gelöst werden.

Das Einengen zur Trockne kann erfindungsgemäß auch durch Sprühtrocknung nach bekannten Verfahren und mit üblichen technischen Geräten, wie Sprühtrockner oder Wirbelschichtgranulatoren erfolgen.

Mit Hilfe der vorliegenden Erfindung werden die Extraktbestandteile, insbesondere die Dianthrone in mikrodisperser Form und/oder in Form einer festen Lösung gebunden. Bei der peroralen Applizierung der Johanniskrautextrakte werden dann unter anderem die Dianthrone in optimaler Weise freigesetzt.

Die Fluidextrakte können auf einfache Weise dadurch hergestellt werden, daß das Johanniskraut in einem geeigneten Lösungsmittel extrahiert wird. Vorzugsweise wird das Lösungsmittel ausgewählt aus Aceton, Chloroform, Ethylacetat und C₁-C₄-Alkoholen, deren Gemischen untereinander und mit Wasser. Besonders bevorzugte Lösungsmittel sind Methanol, Ethanol, und Isopropanol sowie deren wasserfreie oder wasserhaltigen Gemische und insbesondere Ethanol/Wasser-Gemische. Die Extraktion kann in an sich bekannter Form einer Perkolation oder einer mehrstufigen Rühr- oder Wirbelextraktion bei Raumtemperatur oder bei erhöhter Temperatur durchgeführt werden. Je nach dem verwendeten Lösungsmittel weisen die so erhaltenen Rohextrakte einen Spissumgehalt (Trockenrückstand) von etwa 10 bis 20 Gew.-% auf. Neben den genannten Lösungsmitteln ist in gleicher Weise auch die Extraktion mit überkritischen Kohlendioxid für die Herstellung der Rohextrakte einsetzbar.

Unter Zusatz des Trägers wird dann gegebenenfalls unter Erhöhung der Temperatur und/oder unter Vakuum das Lösungsmittel des Extraktes abgezogen und bis auf einen festen oder halbfesten Rückstand zur Trockne eingeengt.

In weiteren bevorzugten Ausführungsformen ist es erfindungsgemäß bevorzugt, die Johanniskrautextrakte mit weiteren Hilfs- und/oder Zusatzstoffen vor dem vollständigen Einengen zur Trockne in Kontakt zu bringen. In gleicher Weise kann es, je nach Auswahl der weiteren Hilfs- und/oder Zusatzstoffe von Vorteil sein, die bereits zur Trockne eingeengten Johanniskrautextrakte mit weiteren Hilfs- und/oder Zusatzstoffen in Kontakt zu bringen. Insbesondere feinteilige Hilfsstoffe, wie Kieselsäure werden vorzugsweise erst nach dem Einengen der Kava-Fluidextrakte mit diesen in Kontakt gebracht.

### Beispiele:

### Beispiel 1:

10 kg Johanniskrautextrakt (Auszugsmittel Ethanol, Gehalt 0,015 % Dianthron, berechnet als Hypericin, wurden mit 3 kg Polyvinylpyrrolidon (Kollidon®25 entsprechend einem K-Wert von 25) versetzt. Nach dem Auflösen des Polyvinylpyrrolidons wurde die alkoholische Phase im Vakuum eingeengt.

Es entstand ein fester Rückstand, der nach dem Zerkleinern mit Magnesiumstearat in Kapseln abgefüllt wurde.

Die Zusammensetzung der Kapsel mit 0,1 mg Dianthron war wie folgt:

| | |
|---|---|
| Rückstand: | 290 mg (= 0,1 mg Dianthron) |
| Magnesiumstearat | 2 mg |

Der Grad der Freisetzung ist in der Tabelle wiedergegeben.

### Beispiel 2:

Analog Beispiel 1 wurde eine Tablette unter Verwendung von Magnesiumstearat und Sorbit hergestellt.

Die Zusammensetzung der Tablette mit 0,1 mg Dianthron war wie folgt:

| | |
|---|---|
| Rückstand | 290 mg (= 0,1 mg Dianthron) |
| Magnesiumstearat | 10 mg |
| Sorbit | 600 mg |

Der Grad der Freisetzung ist in der Tabelle wiedergegeben.

**TABELLE**

| | Vergleich* | Beispiel 1 | Beispiel 3 |
|---|---|---|---|
| Dianthrone | 19,4 % | 98,8 % | 95,5 % |

| | | | |
|---|---|---|---|
| *Als Vergleich wurde eine Kapsel mit 0,1 mg Dianthronen, berechnet als Hypericin, verwendet, die einen herkömmlichen Siccum-Extrakt enthielt. | | | |

### Verfahrensbedingungen:

Entsprechend den Vorschriften des DAB 10 V.5.4 (Wirkstoffreisetzung aus festen peroralen Arzneiformen) wurde unter Einsatz der Blattrührer-Apparatur bei folgenden Bedingungen gearbeitet: 900 ml 0,1 mol/l HCl, 100 U/min, Freisetzungszeit 30 min.

## Patentansprüche

1. Peroral applizierbare Johanniskrautextrakte, dadurch gekennzeichnet, daß sie die nicht flüchtige Phase des Extraktes an Polyvinylpyrrolidon in mikrodisperser Form und/oder in Form einer festen Lösung gebunden, gegebenenfalls neben weiteren Hilfs- und/oder Zusatzstoffen enthalten, wobei das Gewichtsverhältnis von nichtflüchtigen Extraktbestandteilen, insbesondere Spissum oder Siccumphase zu Polyvinylpyrrolidon 1 : 0,4 bis 1 : 5 beträgt.

2. Johanniskrautextrakte nach Anspruch 1, dadurch gekennzeichnet, daß das Polyvinylpyrrolidon einen K-Wert von 12 bis 30 aufweist.

3. Johanniskrautextrakte nach einem oder mehreren der Ansprüche 1 bis 2, dadurch gekennzeichnet, daß das Gewichtsverhältnis von nicht flüchtigen Extraktbestandteilen zu Polyvinylpyrrolidon 1 : 0,7 bis 1 : 1,5 beträgt.

4. Johanniskrautextrakte nach einem oder mehreren der Ansprüche 1 bis 3, dadurch gekennzeichnet, daß die Hilfs- und/oder Zusatzstoffe ausgewählt sind aus Bindemitteln, Gleitmitteln, Füllstoffen, Emulgatoren, Netzmitteln und/oder Sprengmitteln.

5. Verfahren zur Herstellung von peroral applizierbaren Johanniskrautextrakten nach einem oder mehreren der Ansprüche 1 bis 4, dadurch gekennzeichnet, daß man zu einem Johanniskrautextrakt Polyvinylpyrrolidon zugibt und den Fluidextrakt bei gegebenenfalls erhöhter Temperatur und/oder unter Vakuum bis zur Trockne einengt.

6. Verfahren nach Anspruch 5, dadurch gekennzeichnet, daß man einen Johanniskraut-Fluidextrakt aus Aceton, Chloroform, Ethylacetat und C₁-C₄-Alkoholen, insbesondere Methanol, Ethanol und Isopropanol sowie deren Gemische untereinander und mit Wasser einsetzt.

7. Verfahren nach Anspruch 5, dadurch gekennzeichnet, daß den Fluidextrakt durch Sprühtrocknung oder in einem Wirbelschichtgranulator einengt.

8. Verfahren nach einem oder mehreren der Ansprüche 5 bis 7, dadurch gekennzeichnet, daß man die nicht flüchtige Phase eines Johanniskrautextrakt mit weiteren Hilfs- und/oder Zusatzstoffen in Kontakt bringt.

9. Verfahren nach einem oder mehreren der Ansprüche 5 bis 7, dadurch gekennzeichnet, daß man den eingeengten Johanniskrautextrakt mit weiteren Hilfs- und/oder Zusatzstoffen in Kontakt bringt.

## Claims

1. Orally administerable Saint-John's-wort extracts, characterized by containing the non-volatile phase of the extract bound to polyvinyl pyrrolidone in a microdisperse form and/or in the form of a solid solution, optionally in addition to other auxiliaries and/or additives, the weight ratio of non-volatile extract components, especially spissum or siccum phase, to polyvinyl pyrrolidone being from 1:0.4 to 1:5.

2. Saint-John's-wort extracts according to claim 1, characterized in that said polyvinyl pyrrolidone has a K value of from 12 to 30.

3. Saint-John's-wort extracts according to one or more of claims 1 to 2, characterized in that the weight ratio of non-volatile extract components to polyvinyl pyrrolidone is from 1:0.7 to 1:1.5.

4. Saint-John's-wort extracts according to one or more of claims 1 to 3, characterized in that said auxiliaries and/or additives are selected from binders, lubricants, fillers, emulsifiers, wetting agents and/or disintegrants.

5. A process for the preparation of orally administerable Saint-John's-wort extracts according to one or more of claims 1 to 4, characterized in that polyvinyl pyrrolidone is added to a Saint-John's-wort extract, and the fluid extract is concentrated to dryness, optionally at elevated temperature and/or under vacuum.

6. The process according to claim 5, characterized in that a Saint-John's-wort fluid extract from acetone, chloroform, ethyl acetate and C₁-C₄ alcohols, especially methanol, ethanol and isopropanol, and their mixtures with one another and with water is used.

7. The process according to claim 5, characterized in that said fluid extract is concentrated by spray-drying or in a fluidized-bed granulator.

8. The process according to one or more of claims 5 to 7, characterized in that the non-volatile phase of a Saint-John's-wort extract is contacted with further auxiliaries and/or additives.

9. The process according to one or more of claims 5 to 7, characterized in that the Saint-John's-wort extract, after being concentrated, is contacted with further auxiliaries and/or additives.

## Revendications

1. Extraits de millepertuis administrables par voie orale, caractérisés en ce qu'ils contiennent la phase non volatile de l'extrait liée à de la polyvinylpyrrolidone sous forme microdispersée et/ou sous forme de solution solide, éventuellement en plus d'autres substances auxiliaires et/ou complémentaires, le rapport pondéral des composants non volatils de l'extrait, en particulier de la phase spissum (concentrée) ou siccum (sèche), à la Polyvinylpyrrolidone étant de 1:0,4 à 1:5.

2. Extraits de millepertuis selon la revendication 1, caractérisés en ce que la polyvinylpyrrolidone présente une valeur K de 12 à 30.

3. Extraits de millepertuis selon l'une des revendications 1 à 2, ou les deux, caractérisés en ce que le rapport pondéral des composants non volatils de l'extrait à la polyvinylpyrrolidone se situe dans la plage de 1:0,7 à 1:1,5.

4. Extraits de millepertuis selon une ou plusieurs des revendications 1 à 3, caractérisés en ce que les substances auxiliaires et/ou complémentaires sont sélectionnées parmi des agents liants, des agents lubrifiants, des charges, des émulsionnants, des réticulants et/ou des agents de délitage.

5. Procédé de préparation d'extraits de millepertuis administrables par voie orale selon une ou plusieurs des revendications 1 à 4, caractérisé en ce que l'on ajoute à un extrait de millepertuis de la polyvinylpyrrolidone et on concentre l'extrait fluide jusqu'à siccité éventuellement à température élevée et/ou sous vide.

6. Procédé selon la revendication 5, caractérisé en ce qu'on utilise un extrait fluide de millepertuis dans un solvant tel que l'acétone, le chloroforme, l'acétate d'éthyle et des alcools en C₁-C₄, en particulier le méthanol, l'éthanol et l'isopropanol, ainsi que leurs mélanges mutuels et avec de l'eau.

7. Procédé selon la revendication 5, caractérisé en ce que on concentre l'extrait fluide dans un atomiseur ou dans un granulateur en lit fluidisé.

8. Procédé selon une ou plusieurs des revendications 5 à 7, caractérisé en ce qu'on met en contact la phase non volatile d'un extrait de millepertuis avec d'autres substances auxiliaires et/ou complémentaires.

9. Procédé selon une ou plusieurs des revendications 5 à 7, caractérisé en ce qu'on met en contact l'extrait de millepertuis concentré avec d'autres substances auxiliaires et/ou complémentaires.
